(19) Europäisches Patentamt
European Patent Office
Office européen des brevets

(11) Publication number: 0 475 994 B1

(12) # EUROPEAN PATENT SPECIFICATION

(45) Date of publication of patent specification: **14.09.94**

(51) Int. Cl.$^5$: **A61K 31/40**, A61K 31/445

(21) Application number: **90908603.5**

(22) Date of filing: **06.06.90**

(86) International application number:
**PCT/GB90/00866**

(87) International publication number:
**WO 90/14826 (13.12.90 90/28)**

(54) **MACROLIDES FOR THE TREATMENT OF REVERSIBLE OBSTRUCTIVE AIRWAYS DISEASES.**

(30) Priority: **06.06.89 GB 8912935**
**09.04.90 JP 96045/90**

(43) Date of publication of application:
**25.03.92 Bulletin 92/13**

(45) Publication of the grant of the patent:
**14.09.94 Bulletin 94/37**

(84) Designated Contracting States:
**AT BE CH DE DK ES FR GB IT LI LU NL SE**

(56) References cited:
**EP-A- 0 184 162**
**EP-A- 0 315 978**
**EP-A- 0 327 009**

**Immunol. Today, vol. 10, no.1, January 1989, Elsevier Science Publishers Ltd (GB), A.W. Thomson: "FK-505-how much potential?", pages 6-9 see the whole article**

(73) Proprietor: **FISONS plc**
**Fison House**
**Princes Street**
**Ipswich Suffolk IP1 1OH (GB)**

Proprietor: **FUJISAWA PHARMACEUTICAL CO., LTD.**
**4-7, Doshomachi 3-chome**
**Chuo-ku**
**Osaka-shi Osaka 541 (JP)**

(72) Inventor: **NORRIS, Alan, Anthony**
**28 Pitsford Drive**
**Loughborough**
**Leicestershire LE11 0NZ (GB)**
Inventor: **JACKSON, Dale, Michael**
**23 The Green**
**Diseworth**
**Derbyshire DE7 2ON (GB)**
Inventor: **MAKINO, Sohei**
**4-5-14, Midorimachi**
**Mibu-cho**
**Shimotsuga-gun Tochigi 321-02 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**Allergy, vol. 40, 1985, C. Pedersen et al.: "Inhabitory effect of cyclosporin A on histamine release from human leukocytes and rat mast cells", pages 103-107 see the whole article**

**La Médicine Infantile, vol. 93, no.3, November 1986, Maloine S.A. édit., J.L. Menardo et al.: "Le traitement prophylactique de l'asthme, pages 745-753 see page 749, left-hand column, line 53- right-hand column, line 5**

Inventor: **FUKUDA, Takeshi**
**1704-41, Tsuruta**
**Utsunomiya-shi**
**Tochigi 320 (JP)**
Inventor: **AKUTSU, Ikuo**
**4-12-1207, Mutsumimachi**
**Utsunomiya-shi**
**Tochigi 320 (JP)**

(74) Representative: **Wright, Robert Gordon McRae (GB)**
**Fisons plc**
**12 Derby Road**
**GB-Loughborough, Leis LE11 0BB (GB)**

## Description

This invention relates to a novel treatment of reversible obstructive airways disease, more particularly to the use of macrocyclic compounds in the treatment of reversible obstructive airways disease, and to compositions containing such compounds.

European Patent Application 184162 (to Fujisawa Pharmaceuticals Co Ltd) discloses several macrolides (numbered FR-900506, FR-900520, FR-900523 and FR-900525) and derivatives thereof which are isolated from microorganisms belonging to the genus Streptomyces. The macrolides are indicated as immunosuppressive agents. European Patent Application 323042 (to Fisons plc) discloses many macrolides which may be derived from those disclosed in European Patent Application 184162. Again, the compounds are primarily indicated as immunosuppressive agents. European Patent Applications 349049 and 349061 (to Merck & Co Inc, published after the priority date of the present invention) disclose the dihydroxycyclohexyl derivatives of FR-900506 and FR-900520 respectively and indicate them primarily as immunosuppressive agents. None of the documents mentioned above discloses or suggests the use of the compounds disclosed in the treatment of reversible obstructive airways disease.

We have now surprisingly found that a number of macrocyclic compounds, including some of those disclosed in the documents mentioned above (which are herein incorporated by reference), are efficacious in the treatment of reversible obstructive airways disease.

Thus, according to the present invention, we provide the use of a compound of formula I,

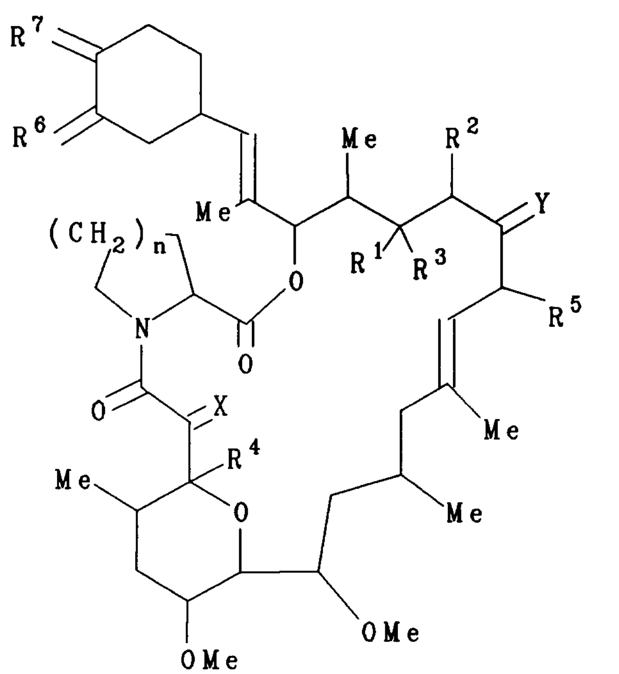

I

wherein $R^1$ and $R^2$ together represent two vicinal hydrogen atoms, or form a second bond between the vicinal carbon atoms to which they are attached;

$R^3$ represents H, OH, $C_{1-10}$ alkoxy or protected hydroxy;

$R^4$ represents OH;

$R^5$ represents allyl, propyl, ethyl or methyl;

$R^6$ and $R^7$ independently represent O, (H,OH), (H,protected hydroxy) or (H,$C_{1-10}$ alkoxy);

X and Y independently represent O or (H,OH);

n is 1 or 2;

or a pharmaceutically acceptable derivative thereof; as active ingredient in the manufacture of a medicament for the treatment of reversible obstructive airways disease.

European Patent Application No 327009 (to Fujisawa Pharmaceuticals Co Ltd, published after the priority date of the present invention) discloses the use of two compounds and their derivatives in the treatment of asthma. Characterising data for the compounds is given, but their structure is not apparent. Should the compounds of European Patent Application No 327009 fall within the scope of formula I above,

they are excluded from the present invention.

Preferably, when $R^3$, $R^6$ and $R^7$ comprise carbon-containing groups, those groups contain from 1 to 6 carbon atoms, eg methyl or methoxy.

Preferably, n is 2.

Desirably, at least one of $R^6$ and $R^7$ represents (H,OH).

We prefer Y to represent O.

The present invention provides the use of all stereoisomers of the compounds of formula I. However, we prefer the compounds of formula I to have the stereochemistry shown in formula Ia:

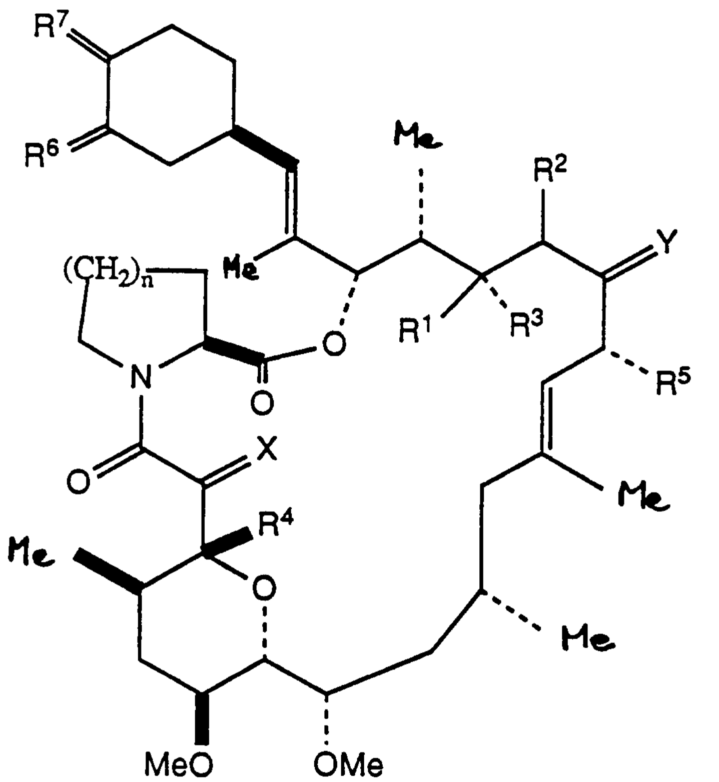

Ia

By the term "protected hydroxy" we mean a group which may be treated so as to yield a hydroxy group. Examples of such groups include an oxygen atom bonded to a protecting group selected from the following:

a) 1-(alkyl C1 to C6 thio)alkyl C1 to C6 such as alkyl C1 to C6 thiomethyl (eg methyl thiomethyl, ethylthiomethyl, propylthiomethyl, isopropylthiomethyl, butylthiomethyl, isobutylthiomethyl, hexylthiomethyl), preferably alkyl C1 to C4 thiomethyl and most preferably methylthiomethyl;

b) trisubstituted silyl such as tri(alkyl C1 to C6)silyl (eg trimethylsilyl, triethylsilyl, tributylsilyl, $^t$butyldimethylsilyl, tri-$^t$butylsilyl), (alkyl C1 to C6)diarylsilyl (eg methyldiphenylsilyl, ethyldiphenylsilyl, propyldiphenylsilyl, $^t$butyldiphenylsilyl), preferably tri(alkyl C1 to C6)silyl and (alkyl C1 to C6)-diphenylsilyl, most preferably $^t$butyldimethylsilyl and $^t$butyldiphenylsilyl; and

c) acyl such as aliphatic acyl, aromatic acyl and aliphatic acyl substituted with aromatic groups, which are derived from carboxylic, sulphonic and carbamic acids.

Preferred protected hydroxy groups that may be mentioned include trialkylsilyloxy groups, for example $^t$butyldimethylsilyloxy.

Further protecting groups and methods for the introduction and removal of protecting groups are described in 'Protective Groups in Organic Chemistry', ed: J W F McOmie, Plenum Press (1973), and 'Protective Groups in Organic Synthesis', T W Greene, Wiley-Interscience (1981).

Pharmaceutically acceptable derivatives of compounds of formula I include esters formed between hydroxy groups and carboxylic acids, and salts (for example alkali metal salts) formed with any acidic groups which may be present.

Specific compounds of formula I which may be mentioned include:

17-allyl-1,14-dihydroxy-12-[2-(4-hydroxy-3-methoxycyclohexyl)-1-methylvinyl]-23,25-dimethoxy-

13,19,21,27-tetramethyl-11,28-dioxa-4-azatricyclo [22.3.1.0$^{4,9}$]octacos-18-ene-2,3,10,16-tetraone,

17-ethyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0$^{4,9}$]octacos-18-ene-2,3,10,16-tetraone,

17-allyl-1,14-dihydroxy-12-[2-(3,4-dihydroxycyclohexyl) -1-methylvinyl]-23,25-dimethoxy-13,19,21,27-tetramethyl-11,28-dioxa-4-asatricyclo[22.3.1.0$^{4,9}$]octacos-18-ene-2,3,10,16-tetraone,

17-ethyl-1,14-dihydroxy-12-[2-(3,4-dihydroxycyclohexyl) -1-methylvinyl]-23,25-dimethoxy-13,19,21,27-tetramethyl-11,28-dioxa-4-azatricyclo[22.3.1.0$^{4,9}$]octacos-18-ene-2,3,10,16-tetraone,

17-propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0$^{4,9}$]octacos-18-ene-2,3,10,16-tetraone,

17-allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0$^{4,9}$]octacos-18-ene-3,10,16-trione, or

17-allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0$^{4,9}$]octacos-18-ene-2,3,10,16-tetraone.

The term "treatment" as used herein includes prophylaxis as well as relieving the symptoms of disease.

The term "reversible obstructive airways disease" will be well understood by those skilled in the art to include conditions such as asthma, including bronchial asthma, allergic asthma, intrinsic asthma, extrinsic asthma and dust asthma, particularly chronic or inveterate asthma (for example late asthma and airway hyper-responsiveness); bronchitis and the like [see for example UK Patent No 2022078 and Brit J Pharmac (1987), 24, 4983-501]. Of particular interest is asthma.

Administration of the active ingredient may be topical (for example by inhalation to the lung), or systemic (for example by oral administration to the gastrointestinal tract).

Dealing first with topical administration, those compounds of formula I which are solids at room temperature may be inhaled as a dry powder which may be pressurized or non-pressurized. In non-pressurized powder compositions, the active ingredient in finely divided form may be used in admixture with a larger sized pharmaceutically acceptable inert carrier comprising particles, eg of up to 100µm diameter. Suitable inert carriers include sugars, for example crystalline lactose. Desirably, at least 95% by weight of the particles of the active ingredient have an effective particle size in the range 0.01 to 10µm.

By providing a large proportion of fine particles of active ingredient the invention enables a lower dosage of drug to be administered and/or for an equivalent amount of drug to produce a greater or longer lasting effect, because fine particles are more likely to penetrate into the deeper regions of the human airways.

The finely divided active ingredient may be made by grinding or milling and is preferably dried thoroughly before formulation.

Non-pressurized powder compositions preferably contain from 0.2 to 5% by weight, more preferably from 0.5 to 2.5% by weight, and particularly from 1 to 1.5% by weight of the active ingredient, and from 95 to 99.8% by weight, more especially from 98.5 to 99% by weight of the carrier.

The composition may alternatively be pressurized and contain a compressed gas, eg nitrogen, or a liquefied gas propellant.

In pressurized compositions, the active ingredient is preferably finely divided, eg having a mass median diameter in the range 0.01 to 10µm (and these finely divided forms of the active ingredient are a feature of the invention). We particularly prefer the active ingredient to have a mass median diameter of less than 4µm and especially of less than 3.0µm and most preferably of less than 2.8µm. We also prefer not more than 5% by weight of the particles to have a diameter of greater than 10µm, and more preferably not less than 90% by weight of the particles to have a diameter of less than 6µm.

We prefer pressurized compositions to contain from 0.01 to 5%, more preferably from 0.1 to 1%, and most preferably from 0.1 to 0.5% of finely divided active ingredient.

By "mass median diameter" we mean that half the particulate mass is in particles of lesser diameter and half in particles of greater diameter than the specified mass median diameter. The mass median diameter is essentially a Stokes diameter and may be determined using a Joyce Loebl sedimentation disc centrifuge either in a two layer or line start photometric mode [Bagness J and Ottaway A; Proc Soc Analyt Chem, Part 4, Vol 9; (1972) pp83-86].

The liquefied propellant medium, and indeed the total composition, is preferably such that the active ingredient does not dissolve therein to any substantial extent.

The liquefied propellant is preferably a gas at room temperature (20°C) and atmospheric pressure, i.e. it should have a boiling point below 20°C at atmospheric pressure. The liquefied propellant should also be non-toxic. Among the suitable liquefied propellants which may be employed are dimethyl ether and alkanes containing up to five carbon atoms, eg butane or pentane, or a lower alkyl chloride, eg methyl, ethyl or

propyl chlorides. The most suitable liquefied propellants are the fluorinated and fluorochlorinated lower alkanes such as are sold under the Registered Trade Mark 'Freon' (the use of the latter type of propellants is a matter of current concern, and they may be replaced by a suitable substitute when such is available). Mixtures of the above mentioned propellants may suitably be employed. Examples of these propellants are:

dichlorodifluoromethane ('Propellant 12'),
1,2-dichlorotetrafluoroethane ('Propellant 114')
trichloromonofluoromethane ('Propellant 11'),
dichloromonofluoromethane ('Propellant 21'),
monochlorodifluoromethane ('Propellant 22'),
trichlorotrifluoroethane ('Propellant 113'), and
monochlorotrifluoromethane ('Propellant 13').

Propellants with improved vapour pressure characteristics may be obtained by using certain mixtures of these compounds, eg propellant 11 with propellant 12, or propellant 12 with propellant 114. For example, propellant 12, which has a vapour pressure of about 570kPa (absolute) at 20°C and propellant 114, with a vapour pressure of about 180kPa (absolute) at 20°C, may be mixed in various proportions to form a propellant having a desired intermediate vapour pressure. We prefer compositions which do not contain trichloromonofluoromethane.

It is desirable that the vapour pressure of the propellant employed be between 380 and 500, and preferably between 410 and 470kPa (absolute) at 20°C. Such a propellant mixture is usable safely with metal containers. Other mixtures of propellant 12 with propellant 114, or of propellant 12 with propellant 11, or of propellant 12 with propellant 11 and propellant 114 with absolute vapour pressures at 20°C in the range 230 to 380 kPa are usable safely with specially reinforced glass containers.

The pressurized composition may also contain a surface active agent. The surface active agent may be a liquid or solid non-ionic surface active agent or may be a solid anionic surface active agent. It is preferred to use the solid anionic surface active agent in the form of the sodium salt.

The preferred solid anionic surface active agent is sodium dioctyl-sulphosuccinate.

The amount of the surface active agent required is related to the solids content of the suspension and to the particle size of the solids. In general it is only necessary to use 5-15%, and preferably 5-8%, of the solid anionic surface active agent by weight of the solids content of the suspension.

When a liquid, non-ionic surface-active agent is employed it should have a hydrophile-lipophile balance (HLB) ratio of less than 10. The HLB ratio is an empirical number which provides a guide to the surface-active properties of a surface-active agent. The lower the HLB ratio, the more lipophilic is the agent, and conversely, the higher the HLB ratio, the more hydrophilic is the agent. The HLB ratio is well known and understood by the colloid chemist and its method of determination is described by W C Griffin in the Journal of the Society of Cosmetic Chemists, Vol 1, No 5, pages 311-326 (1949). Preferably the surface-active agent employed should have an HLB ratio of 1 to 5. It is possible to employ mixtures of surface-active agents, the mixture having an HLB ratio within the prescribed range.

Those surface-active agents which are soluble or dispersible in the propellant are effective. The more propellant-soluble surface-active agents are the most effective.

We prefer the liquid non-ionic surface-active agent to comprise from 0.1 to 2%, and more preferably from 0.2 to 1%, by weight of the total composition. Such compositions tend to be more physically stable on storage.

Among the liquid non-ionic surface-active agents which may be employed are the esters or partial esters of fatty acids containing from 6 to 22 carbon atoms, such as
caproic, octoic, lauric, palmitic, stearic, linoleic, linolenic, oleostearic and oleic acids with an aliphatic polyhydric alcohol or its cyclic anhydride such as, for example, ethylene glycol, glycerol, erythritol, arabitol, mannitol, sorbitol, the hexitol anhydrides derived from sorbitol (the sorbitan esters sold under the Registered Trade Mark 'Span') and the polyoxyethylene and polyoxypropylene derivatives of these esters. Mixed esters, such as mixed or natural glycerides, may be employed. The preferred liquid non-ionic surface-active agents are the oleates of sorbitan, eg those sold under the Registered Trade Marks 'Arlacel C' (Sorbitan sesquioleate), 'Span 80' (Sorbitan monooleate) and 'Span 85' (Sorbitan trioleate). Specific examples of other liquid non-ionic surface-active agents which may be employed are sorbitan monolaurate, polyoxyethylene sorbitol tetraoleate, polyoxyethylene sorbitol pentaoleate, and polyoxypropylene mannitol dioleate.

We particularly prefer compositions containing a sorbitan or sorbitol ester, eg sorbitan trioleate, in a mixture of propellants 12 and 114. We prefer the ratio of propellant 12 to 114 to be in the range from 2:1 to 1:1, and preferably about 1.5:1 by weight, i.e. we prefer an excess of propellant 12 over propellant 114.

We prefer packages containing from about 8 to 30ml of composition, eg a conventional aerosol pressure pack of 10ml. The pack preferably has a valve adapted to deliver unit dosages of between 0.025

and 0.25ml, and preferably 0.05 or 0.1ml, of composition. We prefer the valve to deliver from 2 to 0.02mg, for example 0.2mg of active ingredient and unit doses of these quantities of the drug are provided.

A suitable dose for administration by inhalation is in the range from 0.001 to 0.1mgkg$^{-1}$day$^{-1}$, and preferably 0.01mgkg$^{-1}$day$^{-1}$.

The pressurized compositions of the invention may be made by mixing the various components at a temperature and pressure at which the propellant is in the liquid phase and the active ingredient is in the solid phase.

Thus, according to a second aspect of the present invention, there is provided a method of preparing a pharmaceutical pressurized aerosol composition comprising a compound of formula I, as defined above, or a pharmaceutically acceptable derivative thereof, which comprises mixing the finely divided active ingredient with a pharmaceutically acceptable aerosol propellant.

We further provide a pharmaceutical pressurized aerosol composition comprising a compound of formula I as defined above, or a pharmaceutically acceptable derivative thereof.

In producing the pressurized compositions and packages of the invention, a container equipped with a valve is filled with a propellant containing the finely-divided active ingredient in suspension. A container may first be charged with a weighed amount of dry active ingredient which has been ground to a predetermined particle size, or with a slurry of powder in the cooled liquid propellant. A container may also be filled by introducing powder and propellant by the normal cold filling method, or a slurry of the powder in that component of the propellant which boils above room temperature may be placed in the container, the valve sealed in place, and the balance of the propellant may be introduced by pressure filling through the valve nozzle. As a further alternative a bulk of the total composition may be made and portions of this bulk composition may be filled into the container through the valve. Throughout the preparation of the product care is desirably exercised to minimise the absorption of moisture. On operating the valve, the powder will be dispensed in a stream of propellant, which will vaporise providing an aerosol of dry powder.

Turning now to systemic administration, the active ingredient may be formulated together with known adjuvants, diluents or carriers using conventional techniques to produce tablets or capsules for oral administration to the gastrointestinal tract. Suitable doses for such oral administration are in the range from 0.003 to 0.3mgkg$^{-1}$day$^{-1}$, for example 0.03mgkg$^{-1}$day$^{-1}$.

The method of treatment according to the invention has the advantage that the compounds of formula I as defined above, or pharmaceutically acceptable derivatives thereof, are more efficacious, less toxic, are longer acting, have a broader range of activity, are more potent, produce fewer side effects, are more easily absorbed or have other useful pharmacological properties, than compounds previously used in the treatment of reversible obstructive airways disease.

The dosage to be administered will of course vary with the particular active ingredient, the condition to be treated and with its severity.

It is preferred that the dose be such as to give a sustained rather than a transitory action.

The active ingredient may be administered as divided doses from 1 to 6, and preferably 2 to 4, times per day.

Each dose may comprise 1 or more unit doses.

The invention is illustrated, but in no way limited by, the following Examples.

Example A

Pressurized aerosol composition

| Ingredients | |
|---|---|
| 17-propyl-1-hydroxy-12-[2-(4-hydroxy-3-methoxycyclohexyl)-1-methylvinyl]--23,25-dimethoxy-13,19,21,27-tetramethyl-11,28-dioxa-4-azatricyclo [22.3.1.0$^{4,9}$]octacos-18-ene-2,3,10,16-tetraone (mass median diameter less than 3 microns) | 0.054 |
| Sorbitan trioleate | 0.091 |
| Propellant 114 | 7.099 |
| Propellant 12 | 10.649 |
| | 17.893 |

Method: the sorbitan ester is dispersed in up to half the propellant 12 at -40 ° C while stirring with a high dispersion mixer. The finely divided active ingredient is added to the resulting dispersion and disperses in it. The balance of the propellant 12 is then added at -50 ° C, followed by the propellant 114 also cooled to -50 ° C. The resulting mixtures are then filled into vials onto which valves, eg metering valves, are subsequently crimped.

Example B

Pressurized aerosol composition containing FR-900506

| Ingredients | |
|---|---|
| FR-900506 (mass median diameter less than 3 microns) | 0.054 |
| Sorbitan trioleate | 0.091 |
| Propellant 114 | 7.099 |
| Propellant 12 | 10.649 |
| | 17.893 |

The pressurized aerosol composition was prepared following the method of Example A.

Example C

Assay for inhibitory activity against respiratory resistance and antigen-induced bronchial hyper-responsiveness

Method

**(1) Preparation of inhalation-sensitized guinea pigs**

Male Hartley guinea pigs (weighing about 300g) were each placed in a plastic inhalation chamber. Using an ultrasonic nebulizer (NEU10B, Omron Corporation), an aerosolized solution of ovalbumin in physiological saline ($10mgml^{-1}$) was introduced into the chamber for 10 minutes daily for 10 consecutive days to effect sensitization. The animals were used in the experiments 5 days after establishment of sensitization.

**(2) Pretreatment with FR-900506**

During the period from the first day of sensitization to the day before an antigen challenge, the animals were orally treated with a $1mgml^{-1}$ solution of FR-900506 (in ethanol/olive oil [2:78 v/v]) every other day. Control animals received the ethanol/olive oil vehicle alone in the same manner.

**(3) Experimental schedule**

The experimental period was 5 days from day 1 to day 5, and the inhalation challenge with the antigen was given on day 2.

On day 1, and 30 minutes before antigen inhalation challenge on day 2, metopirone (an endogenous cortisol synthesis inhibitor) was intravenously administered ($10mgkg^{-1}$). So that the animals could tolerate the antigen at comparatively high concentrations, chlorpheniramine maleate, an antihistaminic, was intraperitoneally administered ($10mgkg^{-1}$) following the second dose of metopirone. After the pretreatment discussed above, each guinea pig was transferred to an animal box connected to an oscillator and fixed therein with its head projecting out. The head was then covered with an aerochamber communicating with a Devilvis 646 nebulizer.

**(4) Assay for respiratory resistance**

The assay for respiratory resistance was performed by the oscillation method of Mead et al with some modification (Allergy, 37, 10, 980-991, 1988). The antigen inhalation challenge was made by nebulizing a

saline solution of ovalbumin (20mgml$^{-1}$) with 51min$^{-1}$ of air and causing the animals to inhale for 1 minute. The results are shown in Table 1.

**(5) Assay for antigen-induced bronchial hyper-responsiveness to acetylcholine**

Guinea pigs prepared by the method described above were placed in an animal box as described above and the baseline respiratory resistance was measured. The animals were then caused to inhale a nebulized saline solution of acetylcholine (in an ascending concentration series of 156 to 5000μgml$^{-1}$) for 1 minute at each concentration until the respiratory resistance was increased to twice the baseline value. From the concentration-resistance curve constructed from the acetylcholine concentration and respiratory resistance data, the acetylcholine concentration necessary for increasing the respiratory resistance to twice the baseline value [ie $PC_{200}$-Ach (μgml$^{-1}$)] was calculated. The results are shown in Table 2.

**(6) Analysis of data**

The results are expressed as mean ± SEM. Student's t-test was used as the test for significant difference.

Results

Table 1: Inhibitory effect of FR-900506 on respiratory resistance (%)

| Time after antigen inhalation challenge (hrs) | Change in respiratory resistance (%) Control (n=15) | FR-900506 treated (n=7) |
|---|---|---|
| 3 | 131 ± 10 | *95 ± 7.3 |
| 6 | 185 ± 18 | *115 ± 9.5 |
| 9 | 152 ± 14 | *108 ± 4.3 |

*P<0.05

Change in respiratory resistance (%)

$$= \frac{\text{Respiratory resistance after challenge}}{\text{Respiratory resistance before challenge}} \times 100\%$$

Table 2: Inhibitory effect of FR-900506 on antigen induced hyper-responsiveness to acetylcholine

| Time after antigen inhalation challenge (hrs) | $PC_{200}$-Ach ($\mu gml^{-1}$) Control (n=15) | FR-900506 treated (n=7) |
|---|---|---|
| Before challenge | 1866 ± 313 | 1192 ± 358 |
| 24 | 553 ± 80 | 883 ± 191 |
| 72 | 1353 ± 196 | 1492 ± 354 |

Control: Before challenge vs 24: * ; 24 vs 72: * ; Before challenge vs 72: NS
FR-900506 treated: Before challenge vs 24: NS ; 24 vs 72: NS ; Before challenge vs 72: NS

*P<0.001

NS = no significant difference

The results indicate that the compounds of formula I are likely to be most efficacious in the treatment of reversible obstructive airways disease.

Example D

Acute toxicity of FR-900506

An acute intraperitoneal toxicity study of FR-900506 in ddy mice revealed no deaths at $100mgkg^{-1}$

## Claims

**Claims for the following Contracting States : AT, BE, CH/LI, DE, DK, FR, GB, IT, LU, NL, SE**

**1.** The use of a compound of formula I,

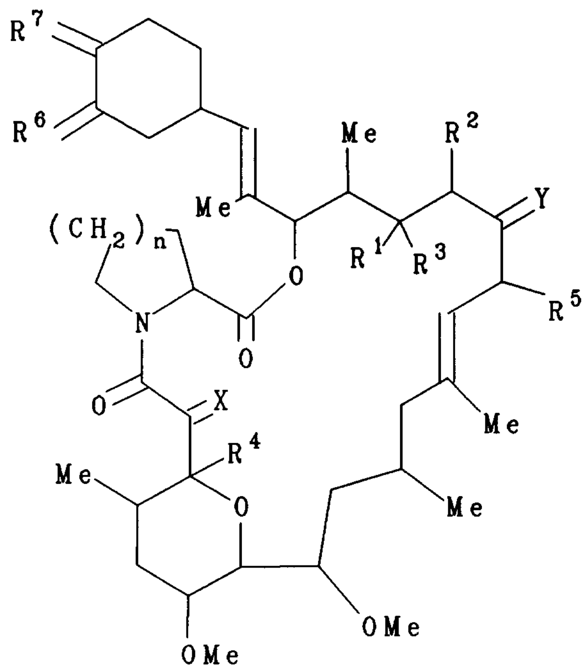

I

wherein $R^1$ and $R^2$ together represent two vicinal hydrogen atoms, or form a second bond between the vicinal carbon atoms to which they are attached;

$R^3$ represents H, OH, $C_{1-10}$ alkoxy or protected hydroxy;

$R^4$ represents OH;

$R^5$ represents allyl, propyl, ethyl or methyl;

$R^6$ and $R^7$ independently represent O, (H,OH), (H,protected hydroxy) or (H,$C_{1-10}$ alkoxy);

X and Y independently represent O or (H,OH);

n is 1 or 2;

or a pharmaceutically acceptable derivative thereof;

as active ingredient in the manufacture of a medicament for the treatment of reversible obstructive airways disease.

**2.** The use according to claim 1, wherein at least one of $R^6$ and $R^7$ represents (H,OH).

**3.** The use according to claim 1 or claim 2, wherein Y represents O.

**4.** The use according to any one of the preceding claims, wherein n is 2.

**5.** The use according to claim 1, wherein the compound of formula I is:

17-ethyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.$0^{4,9}$]octacos-18-ene-2,3,10,16-tetraone,

17-allyl-1,14-dihydroxy-12-[2-(3,4-dihydroxycyclohexyl) -1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone,

17-ethyl-1,14-dihydroxy-12-[2-(3,4-dihydroxycyclohexyl) -1-methylvinyl]-23,25-dimethoxy-

13,19,21,27-tetramethyl-11,28-dioxa-4-azatricyclo[22.3.1.$0^{4,9}$]octacos-18-ene-2,3,10,16-tetraone,
17-propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.$0^{4,9}$]octacos-18-ene-2,3,10,16-tetraone,
17-allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.$0^{4,9}$]octacos-18-ene-3,10,16-trione, or
17-allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.$0^{4,9}$]octacos-18-ene-2,3,10,16-tetraone.

**6.** The use according to claim 1, wherein the compound of formula I is:
17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.$0^{4,9}$]octacos-18-ene-2,3,10,16-tetraone.

**7.** A pharmaceutical pressurized aerosol composition comprising a compound of formula I as defined in any one of the preceding claims, or a pharmaceutically acceptable derivative thereof, and a pharmaceutically acceptable aerosol propellant.

**8.** A method of preparing a pharmaceutical aerosol composition comprising a compound of formula I as defined in any one of claims 1 to 6, or a pharmaceutically acceptable derivative thereof, which comprises mixing the finely divided active ingredient with a pharmaceutically acceptable aerosol propellant.

**9.** The use according to any one of claims 1 to 6, wherein the disease is asthma.

**Claims for the following Contracting State : ES**

**1.** A method of preparing a pharmaceutical aerosol composition comprising a compound of formula I,

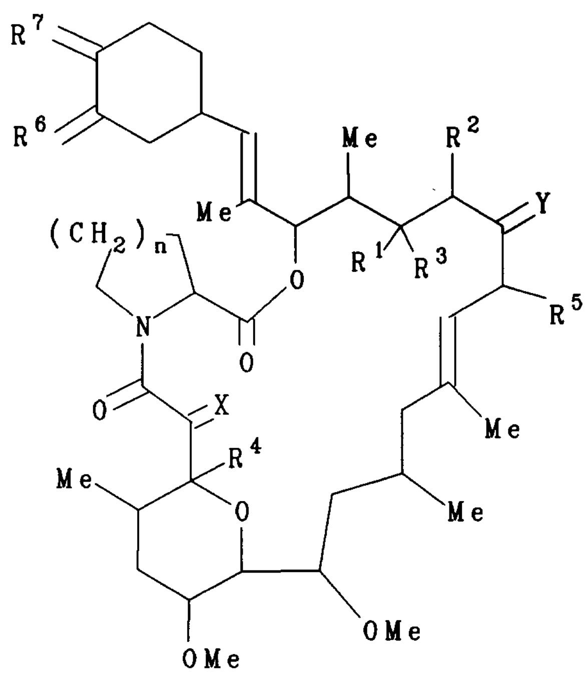

I

wherein $R^1$ and $R^2$ together represent two vicinal hydrogen atoms, or form a second bond between the vicinal carbon atoms to which they are attached;
$R^3$ represents H, OH, $C_{1-10}$ alkoxy or protected hydroxy;
$R^4$ represents OH;
$R^5$ represents allyl, propyl, ethyl or methyl;
$R^6$ and $R^7$ independently represent O, (H,OH), (H,protected hydroxy) or (H,$C_{1-10}$ alkoxy);
X and Y independently represent O or (H,OH);
n is 1 or 2;

or a pharmaceutically acceptable derivative thereof;

which comprises mixing the finely divided active ingredient with a pharmaceutically acceptable aerosol propellant.

2. The method according to claim 1, wherein at least one of $R^6$ and $R^7$ represents (H,OH).

3. The method according to claim 1 or claim 2, wherein Y represents O.

4. The method according to any one of the preceding claims, wherein n is 2.

5. The method according to claim 1, wherein the compound of formula I is:

17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone,

17-ethyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone,

17-allyl-1,14-dihydroxy-12-[2-(3,4-dihydroxycyclohexyl)-1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone,

17-ethyl-1,14-dihydroxy-12-[2-(3,4-dihydroxycyclohexyl)-1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone,

17-propyl-1-hydroxy-12-[2(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone,

17-allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-ene-3,10,16-trione, or

17-allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone.

**Patentansprüche**

**Patentansprüche für folgende Vertragsstaaten : AT, BE, CH, LI, DE, DK, FR, GB, IT, LU, NL, SE**

1. Verwendung einer Verbindung der Formel (I)

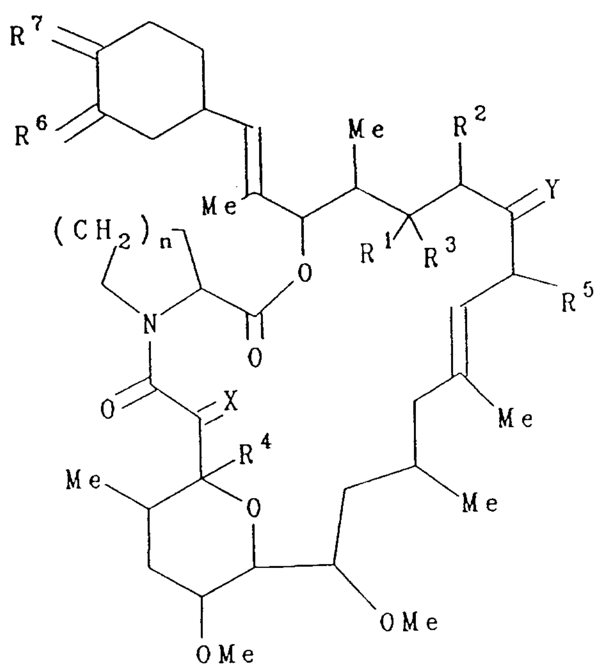

(I),

worin $R^1$ und $R^2$ zusammen zwei benachbarte Wasserstoffatome darstellen, oder eine zweite Bindung zwischen den benachbarten Kohlenstoffatomen, an die sie gebunden sind, bilden; $R^3$ H, OH, $C_1$-$C_{10}$-Alkoxy oder geschütztes Hydroxy ist; $R^4$ die Bedeutung OH hat; $R^5$ Allyl, Propyl, Ethyl oder Methyl darstellt; $R^6$ und $R^7$ unabhängig O, (H,OH), (H,geschütztes Hydroxy) oder (H,$C_1$-$C_{10}$-Alkoxy) bedeuten;

X und Y unabhängig O oder (H,OH) sind; n 1 oder 2 ist; oder eines pharmazeutisch annehmbaren Derivats hievon; als aktiver Bestandteil bei der Herstellung eines Medikaments zur Behandlung reversibler obstruktiver Atemwegserkrankungen.

**2.** Verwendung nach Anspruch 1, wobei zumindest eines von $R^6$ und $R^7$ (H,OH) darstellt.

**3.** Verwendung nach Anspruch 1 oder 2, wobei Y die Bedeutung O hat.

**4.** Verwendung nach einem der vorhergehenden Ansprüche, wobei n 2 ist.

**5.** Verwendung nach Anspruch 1, wobei die Verbindung der Formel (I) ist:
17-Ethyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,
17-Allyl-1,14-dihydroxy-12-[2-(3,4-dihydroxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,
17-Ethyl-1,14-dihydroxy-12-[2-(3,4-dihydroxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,
17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,
17-Allyl-1,2,14-trihydroxy-12-[2-(4-hydroxy-3-methoxycyclo-hexyl)-1-methylvinyl]-23,25-dimethoxy-13,19,21,27-tetramethyl-11,28-dioxa-4-azatricyclo[22.3.1.$0^{4,9}$]octacos-18-en-3,10,16-trion, oder
17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon.

**6.** Verwendung nach Anspruch 1, wobei die Verbindung der Formel (I) ist:
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon.

**7.** Pharmazeutische unter Druck stehende Aerosol-Zusammensetzung, welche eine Verbindung der Formel (I), wie in einem der vorhergehenden Ansprüche definiert, oder ein pharmazeutisch annehmbares Derivat hievon, und ein pharmazeutisch annehmbares Aerosol-Treibmittel umfaßt.

**8.** Verfahren zur Herstellung einer pharmazeutischen Aerosol-Zusammensetzung, die eine Verbindung der Formel (I), wie in einem der Ansprüche 1 bis 6 definiert, oder ein pharmazeutisch annehmbares Derivat hievon, umfaßt, welches Verfahren das Mischen des fein zerteilten aktiven Bestandteils mit einem pharmazeutisch annehmbaren Aerosol-Treibmittel umfaßt.

**9.** Verwendung nach einem der Ansprüche 1 bis 6, wobei die Erkrankung Asthma ist.

**Patentansprüche für folgenden Vertragsstaat : ES**

**1.** Verfahren zur Herstellung einer pharmazeutischen Aerosol-Zusammensetzung, die eine Verbindung der Formel (I)

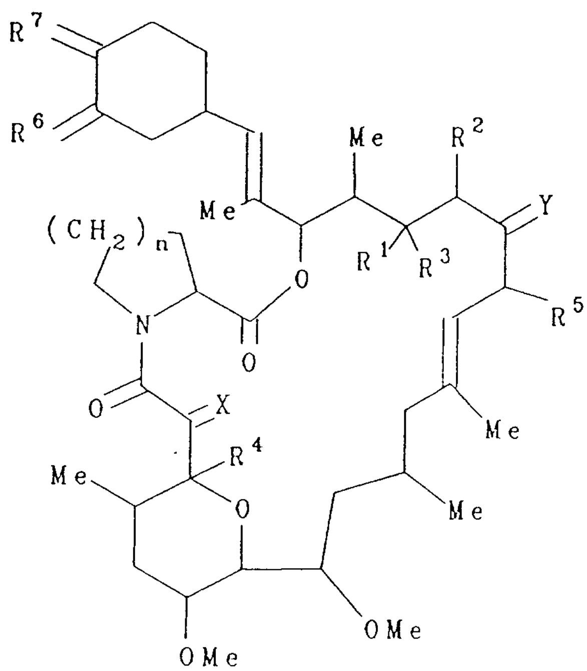

(I)

worin $R^1$ und $R^2$ zusammen zwei benachbarte Wasserstoffatome darstellen, oder eine zweite Bindung zwischen den benachbarten Kohlenstoffatomen, an die sie gebunden sind, bilden; $R^3$ H, OH, $C_1$-$C_{10}$-Alkoxy oder geschütztes Hydroxy ist; $R^4$ die Bedeutung OH hat; $R^5$ Allyl, Propyl, Ethyl oder Methyl darstellt; $R^6$ und $R^7$ unabhängig O, (H,OH), (H,geschütztes Hydroxy) oder (H,$C_1$-$C_{10}$-Alkoxy) bedeuten; X und Y unabhängig O oder (H,OH) sind; n 1 oder 2 ist; oder ein pharmazeutisch annehmbares Derivat hievon umfaßt; welches Verfahren das Mischen des fein zerteilten aktiven Bestandteils mit einem pharmazeutisch annehmbaren Aerosol-Treibmittel umfaßt.

**2.** Verfahren nach Anspruch 1, wobei zumindest eines von $R^6$ und $R^7$ (H,OH) darstellt.

**3.** Verfahren nach Anspruch 1 oder 2, wobei Y die Bedeutung O hat.

**4.** Verfahren nach einem der vorhergehenden Ansprüche, wobei n 2 ist.

**5.** Verfahren nach Anspruch 1, wobei die Verbindung der Formel (I) ist:

17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,

17-Ethyl-1,14-dihydroxy-12-(2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,

17-Allyl-1,14-dihydroxy-12-[2-(3,4-dihydroxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,

17-Ethyl-1,14-dihydroxy-12-[2-(3,4-dihydroxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,

17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon,

17-Allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-3,10,16-trion, oder

17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-en-2,3,10,16-tetraon.

**Revendications**

**Revendications pour les Etats contractants suivants : AT, BE, CH, DE, DK, FR, GB, IT, LI, LU, NL, SE**

1. Utilisation d'un composé de formule (I) :

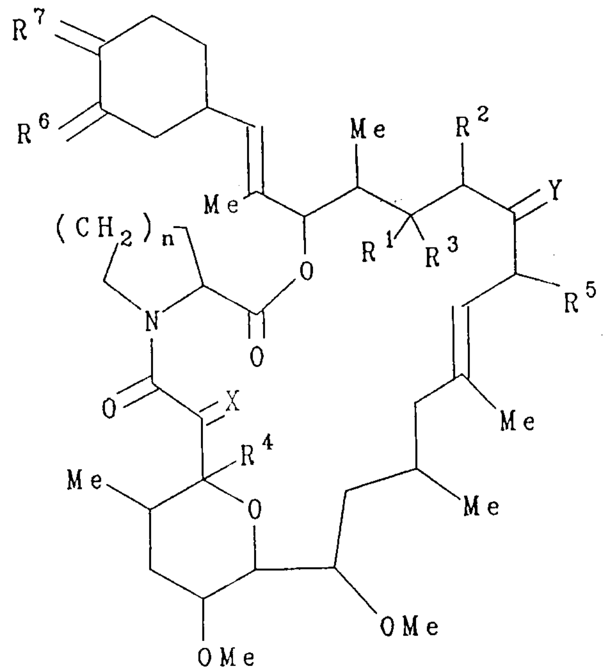

(I)

dans lequel

$R^1$ et $R^2$ représentent conjointement deux atomes d'hydrogène vicinaux ou forment une deuxième liaison entre les atomes de carbone vicinaux auxquels ils sont attachés;

$R^3$ représente H, OH, alcoxy en $C_{1-10}$ ou hydroxyle protégé;

$R^4$ représente OH;

$R^5$ représente allyle, propyle, éthyle ou méthyle;

$R^6$ et $R^7$ représentent indépendamment O, (H, OH), (H, hydroxyle protégé) ou (H, alcoxy en $C_{1-10}$);

X et Y représentent indépendamment O ou (H, OH), et

n est 1 ou 2;

ou un dérivé pharmaceutiquement acceptable de celui-ci, comme ingrédient actif dans la préparation d'un médicament pour le traitement de maladies des voies respiratoires, obstructives, réversibles.

2. Utilisation suivant la revendication 1, dans laquelle au moins un des $R^6$ et $R^7$ représente (H, OH).

3. Utilisation suivant la revendication 1 ou 2, dans laquelle Y représente O.

4. Utilisation suivant l'une quelconque des revendications précédentes, dans laquelle n est 2.

5. Utilisation suivant la revendication 1, dans laquelle le composé de formule (I) est :

la 17-éthy1-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,

la 17-allyl-1,14-dihydroxy-12-[2-(3,4-dihydroxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,

la 17-éthyl-1,14-dihydroxy-12-[2-(3,4-dihydroxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,

la 17-propyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,

la 17-allyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-3,10,16-trione, ou

la 17-allyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone.

**6.** Utilisation suivant la revendication 1, dans laquelle le composé de formule (I) est :
la 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone.

**7.** Composition pressurisée pharmaceutique d'un aérosol comprenant un composé de formule (I) tel que défini dans l'une quelconque des revendications précédentes, ou un dérivé pharmaceutiquement acceptable de celui-ci, et un propulseur d'aérosol pharmaceutiquement acceptable.

**8.** Procédé de préparation d'une composition pharmaceutique d'un aérosol comprenant un composé de formule (I) tel que défini dans l'une quelconque des revendications 1 à 6, ou un dérivé pharmaceutiquement acceptable de celui-ci, qui comprend le mélange de l'ingrédient actif finement divisé avec un propulseur d'aérosol pharmaceutiquement acceptable.

**9.** Utilisation suivant l'une quelconque des revendications 1 à 6, dans laquelle la maladie est l'asthme.

**Revendications pour l'Etat contractant suivant : ES**

**1.** Procédé de préparation d'une composition pharmaceutique d'un aérosol comprenant un composé de formule (I) :

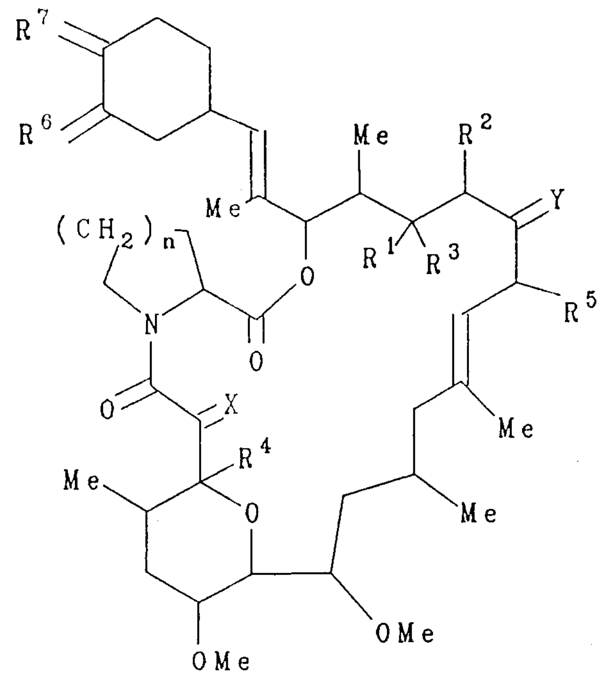

(I)

dans lequel

$R^1$ et $R^2$ représentent conjointement deux atomes d'hydrogène vicinaux ou forment une deuxième liaison entre les atomes de carbone vicinaux auxquels ils sont attachés;

$R^3$ représente H, OH, alcoxy en $C_{1-10}$ ou hydroxyle protégé;

$R^4$ représente OH;

$R^5$ représente allyle, propyle, éthyle ou méthyle;

$R^6$ et $R^7$ représentent indépendamment O, (H, OH), (H, hydroxyle protégé) ou (H, alcoxy en $C_{1-10}$);

X et Y représentent indépendamment O ou (H, OH), et

n est 1 ou 2;

ou un dérivé pharmaceutiquement acceptable de celui-ci, qui comprend le mélange de l'ingrédient actif finement divisé avec un propulseur d'aérosol pharmaceutiquement acceptable.

**2.** Procédé suivant la revendication 1, dans lequel au moins un des $R^6$ et $R^7$ représente (H, OH).

**3.** Procédé suivant la revendication 1 ou 2, dans lequel Y représente O.

**4.** Procédé suivant l'une quelconque des revendications précédentes, dans lequel n est 2.

**5.** Procédé suivant la revendication 1, dans lequel le composé de formule (I) est :
la 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone;
la 17-éthyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,
la 17-allyl-1,14-dihydroxy-12-[2-(3,4-dihydroxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,
la 17-éthyl-1,14-dihydroxy-12-[2-(3,4-dihydroxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,
la 17-propyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone,
la 17-allyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-3,10,16-trione, ou
la 17-allyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.$0^{4,9}$]octacos-18-ène-2,3,10,16-tétraone.